# EUROPEAN PATENT APPLICATION

(11) **EP 2 468 184 A1**
(43) Date of publication of application: **27.06.2012**
(21) Application number: 10196986.3
(22) Date of filing: 24.12.2010
(51) Int. Cl.: A61B 5/103

(54) **Method and system for processing load data comprising the load measured on a lower extremity of a patient**

(71) Applicant: Evalan BV, 1314 AN Almere (NL); UMC Utrecht Holding B.V., 3584 CM Utrecht (NL); ACADEMISCH ZIEKENHUIS GRONINGEN, 9713 GZ Groningen (NL); Rijksuniversiteit Groningen, 9712 CP Groningen (NL)
(72) Inventor: Vrij, Karin Elizabeth, 3571 PM, Utrecht (NL); van Merkerk, Rutger Olof, 3582 ST, Utrecht (NL); Schwietert, Hendrik Reinier, 1019 RP, Amsterdam (NL); Holtslag, Herman, 1391 BE, Abcoude (NL)
(74) Representative: Land, Addick Adrianus Gosling

(57) **Abstract**

Method for processing load data comprising the load measured on a lower extremity of a patient suffering from, but not exclusively, lower extremity injury, in particular a lower extremity bone injury such as a fracture, over a time period during or before rehabilitation, the method comprising selecting dynamic load data from said load data, wherein load data is selected as dynamic load data when the load temporary, within a predetermined load time period, exceeds a predetermined load threshold.

## Description

The present invention relates to a method for processing measurements of the load on a lower extremity of a patient suffering from lower extremity injury, in particular a lower extremity bone injury such as a fracture, over a time period during or before rehabilitation. The invention further relates to a system for establishing at least one parameter being indicative for the rehabilitation progress of a patient suffering from lower extremity injury, in particular a lower extremity bone injury such as a fracture.

Bone is a living, dynamic and metabolic active tissue in which growth, remodelling and fracture healing take place. These three processes are all stimulated by biomechanical and biophysical signals. Bone remodelling is a constant process, whereby bone is strengthened in the direction of the applied stress. Due to this process, bone can adapt its structure to the demand of the mechanical conditions. Bone remodelling corresponds to Wolff's law, which was proposed more than 100 years ago, and which postulates that the thickness and number of trabeculae correspond to the quantitative distribution of mechanical stresses.

Each year thousands of people suffer a lower extremity fracture. Complications such as delayed healing and non-unions (fractures that do not heal in time or do not heal at all) occur in 5 to 10% of the patients that have incurred a lower extremity fracture. The associated costs of these complications for society and for the individual patient are high. One of the factors that contribute to bone healing is mechanical loading. Under- or overloading during the rehabilitation could cause the occurrence of delayed healing or non-union. The distribution of loading and the amount of exercise by the patient during the rehabilitation also affect the healing process.

Therefore, a goal of the present invention is, amongst other goals, to provide an efficient method for processing measurements of load on a lower extremity of a patient suffering from lower extremity injury for instance to establish parameters of which at least one parameter being indicative for the rehabilitation progress of a patient suffering from lower extremity injury.

The above goal is met by the present invention, amongst other goals, by a method for processing load data comprising the load measured on a lower extremity of a patient suffering from lower extremity injury, in particular a lower extremity bone injury such as a fracture, over a time period during or before rehabilitation, the method comprising selecting dynamic load data from said load data, wherein load data is selected as dynamic load data when the load temporary, within a predetermined load time period, exceeds a predetermined load threshold. According to the invention, dynamic load data is selected from the load data, as dynamic load data is considered relevant data for assessing the progress of rehabilitation. Load data, for instance in the form of pressure (Pa) or load (N) per time unit (s), is determined as being dynamic load in case the load is temporary, thus not exceeding the predetermined load time period, higher than the load threshold.

Only selecting dynamic load data from the load data reduces the needed bandwidth, in case the load date needs to be transmitted, and/or data storage capacity, in case the data needs to be processed further at a later stage. Also the further processing is easier due to the reduced, in particular condensed, data.

According to a preferred embodiment the load time period is 0,01 to 20 seconds, preferably 0,02 to 10 seconds, more preferably 0,075 to 5 seconds and even more preferably the load time period is 0,1 to 2 seconds. This period is chosen such that it encapsulates the relationship between bone growth and dynamic stresses and strains, where the bone growth is stimulated if the stresses and strains last at least a minimum period of time, but is not stimulated if they remain at a constant value longer than a maximum period of time.

According to a further preferred embodiment the load threshold is at least 3%, more preferable at least 10%, even more preferably at least 20% of the total body weight of the patient. This threshold is chosen such that it captures the relationship between bone growth and loading, where the bone growth is not stimulated if the stress does not exceed at least a minimum value.

A method according to a further preferred embodiment further comprises increasing the load threshold with progressing time in the load data, in particular with rehabilitation progression. This increase in load threshold is chosen such that it reflects that, as the bone heals, the stress and strain levels that are necessary for stimulation of bone growth increases over time during the rehabilitation process.

According to a further preferred embodiment of the method according to the invention, said load data comprises axial load data having an axial component with respect to the lower extremity. Substantially axially oriented forces or loads are measured for obtaining the load data, as from these loads a reliable indication of the progress of the rehabilitation can be determined. According to the invention, axially oriented forces or loads in the lower extremity, in particularly in the injured bone thereof, and more in particular dynamic changes thereof, allow determining a reliable indication of the progress.

According to a further preferred embodiment the load data comprises pressure measurements under the foot, in particular under the heel to measure the axial component of the load of said lower extremity. A suitable pressure sensor can be used for measuring the pressure. Pressure sensors for measuring pressure under the foot, in particular the heel, as such are known.

It is possible to first measure the load data, for instance using the pressure sensor, and subsequently store the measured data. The load data can then be processed according to the method according to the invention. However, according to a further preferred embodiment, the method further comprises the step of measuring the load on said lower extremity for providing the load data. The load data is then measured real time and preferably the dynamic load data is selected at least substantially real time from the measured load data for further processing.

Preferably the steps of measuring and processing the load data take place in at least one portable device, preferably carried by the patient, and wherein the method preferably further comprises the step of sending, preferably wirelessly, the dynamic load data to a remote server. Only transmitting the dynamic load data, instead of transmitting all load data, reduces the required bandwidth capacity for the portable device. The remote server is hereby arranged to receive the data, for instance over the internet. Said server is provided with suitable storage and processing means for further processing and/or storing the received data. Also in case the dynamic load data is stored in data storage means of the portable device for offline download to for instance a (remote) server, more data can be stored, allowing a longer period of measuring.

It should however be noted that although the invention is disclosed for processing load data on a lower extremity, i.e. a leg, the method could also be used for processing load data from other parts of the human or animal body. For instance, the same load data obtained from for instance the foot can be used to determine loads in the spine.

According to a further preferred embodiment of the method according to the invention, selecting dynamic load data comprises identifying instances of dynamic loading in the load data as clinically relevant data, wherein a load peak in the load data is identified as an instance of dynamic loading if the load peak exceeds the load threshold for a time period within the load time period. By defining instances of dynamic loading from the load data, the data is efficiently quantified. This allows a physician to efficiently determine the progress of rehabilitation progress inter alia based on the identified instances of dynamic loading.

Preferably a second load peak following a first load peak identified as an instance of dynamic loading is identified as a subsequent instance of dynamic loading if:
- the minimum load between the first and second peaks is lower than a predetermined differentiating threshold, wherein the differentiating threshold is preferably 3%, more preferably 10% of the total body weight of the patient, or;
- the difference between the minimum load between the first and second peak and each of the maximum loads of the first and second peaks is larger than the load threshold.

This allows further distinguishing clinical relevant instances of dynamic loading from the load data. It is discovered that not only the absolute amplitude of the load is relevant, but also the difference in loads between two subsequent peaks. These additional identified instances of dynamic loading allow a physician to determine the progress of rehabilitation more reliable.

The invention furthermore relates to a method for establishing at least one parameter being indicative for the rehabilitation progress of a patient suffering from lower extremity injury, in particular a lower extremity bone injury such as a fracture, the method comprising the steps of:
- processing load data according to the method according to the invention;
- establishing said parameter based on the identified instances of dynamic loading, wherein the parameter is based on the number of instances of dynamic loading, the average load, preferably the average peak load, of the instances of dynamic loading, the total duration of the instances of dynamic loading, preferably the total duration of the load exceeding the load threshold, or a combination thereof.

The established parameter can be used by a physician to determine the progress of rehabilitation. The method is hereby a non-diagnostic method; i.e. a physician is always needed to determine the progress. The established parameter by the method according to the invention can be used, amongst other data, by a physician for determining the progress.

In order to facilitate the physician in this progress, parameters are established from the identified instances of dynamic loading, these parameters being indicative of the progress of rehabilitation.

A preferred embodiment of the method comprises identifying active loading time periods and resting time periods from the dynamic load data, the active loading time periods being representative of a period of activity of the patient, in particular having more instances of dynamic loading per time period than the resting time periods being representative of a period of resting of the patient, wherein the parameter is established based on the number of active loading time periods followed by a resting time period. In particular periods of high activity followed by a period of relative low activity appear to stimulate bone growth. By determining the number of active periods followed by non-active periods, this number can be used as an indication for the progress of said bone growth.

An active loading time period may be identified when the frequency of instances of dynamic loading during a predetermined time period is higher than a predetermined threshold. A resting time period may be identified when the total number of instances of dynamic loading is below said predetermined threshold for a predetermined time period.

A further preferred embodiment comprises the step of outputting at least one parameter to the patient and/or a physician for providing feedback on the rehabilitation progress. This allows feedback to for instance the patient. The patient can adapt his activity to stimulate the rehabilitation, for instance bone growth. By providing a parameter to the physician, also the physician can monitor the progress. Preferably the step of outputting comprises the step of making available the parameter and/or the, preferably dynamic, load data. Said data can for instance be made available online by the remote server as mentioned above via internet.

The invention furthermore relates to a system for establishing at least one parameter being indicative for the rehabilitation progress of a patient suffering from lower extremity injury, in particular a lower extremity bone injury such as a fracture, the system comprising:
- a load measuring device arranged for measuring load data comprising the load measured on the lower extremity of the patient over a time period during or before rehabilitation;
- a processing device for processing the load data measured by the load measuring device into dynamic load data according to the invention, and;
- an analysing device for establishing at least one parameter according to the invention.

The load measuring device, the processing device and the analysing device may be formed integrally and may for instance be carried by the patient. It is however preferred that the load measuring device and the processing device are formed integrally and are arranged to be carried by a patient. The analysing step may then be performed in a separate device, for instance a remote server.

A preferred embodiment of the system according further comprises a remote server, wherein at least one of the load measuring device, the processing device and the analyzing device is arranged to send the data and/or the parameter to the remote server, preferably wirelessly. This embodiment includes a feedback system that is able to provide adequate feedback to patient and or physician.

The present invention is further illustrated by the following Figures and Example, which show a preferred embodiment of the method according to the invention, and are not intended to limit the scope of the invention in any way, wherein:
- Figure 1 shows interpretation of IDL identification in case the loading does not fall under a minimum threshold (for example 10% of the Body Weight of the patient), wherein the Y-axis represents the loading relative to the body weight and the X-axis represents time in seconds;
- Figure 2 shows interpretation of maximum pressure (amplitude) and duration, wherein the Y-axis represents the loading relative to the body weight and the X-axis represents time in seconds, and;
- Figure 3 - 10 show examples of load data for 8 different activities, wherein the Y-axis represents the loading relative to the body weight and the X-axis represents time in seconds.

Measurement of the load that a patient exerts on his lower extremity during rehabilitation is established by using an in-shoe pressure sensor. In this example, this is a thin sensor, consisting of one or more thin plates that deform with increasing pressure, and strain gauges that measure the deformation of the plate(s). This sensor could be placed in the shoe, under the heel of the foot, as the axial pressure is the most relevant during rehabilitation of lower extremity fractures.

Measurement data from this sensor could be collected by a small device, a sensor server, that is carried by the patient, and that transmits this data wirelessly to a central computer for storage and processing. This sensor server could be outfitted with a screen, lights, a speaker and other components that could be used to provide feedback messages or alarms to the patient of various kinds. This sensor server could process or partially process the measurement data and calculate parameters that are indicative of the progress of rehabilitation of the lower extremity of the patient.

In the process of fracture repair mechanical forces induce osteogenesis, which has a positive influence on the mass and distribution of callus, on bone hypertrophy, on the formation of fibrous connective tissue, on gene expression, on the mineral content, on the intramembranous tissue, on bone formation and on bone strength. When a fracture is compressed and deformed beyond a certain threshold, the hydrodynamic load generates an electrophysiological response in the form of electric streaming potentials. The negative potential activates the osteoblasts in the fracture side, and the positive potential the osteoclasts. It is possible that streaming potentials, in combination with fluid flow in the canaliculi, activate mechanotransduction, bone response to mechanical loading, and induce osteogenesis. During this process mechanosensitive cells (osteocytes) sense mechanical stimuli and transform this to electrical or biochemical signals. It occurs if the stimuli exceed a certain threshold. This mechanism of mechanotransduction with the associated electrophysiological response results in the observation that dynamic movements rather than static forces enhance fracture healing and bone remodelling, as each loading cycle results in an activation of this mechanism. Thus, variation between bouts of dynamic movements and bouts of rest are required for fracture healing.

The timing of mechanical loading is important. Early weight bearing in the course of fracture treatment seems to be beneficial, because this period is particularly sensitive to mechanical stimuli. The clear advantage of early weight bearing is that early mobilisation increases the bone turnover metabolism: it stimulates callus and bone growth in contrast to non weight bearing, which leads to bone resorption.

Based on these biomechanical principles the present invention could use four parameters that together could be used as an indication for the rehabilitation progression of patients with lower extremity fractures. They are derived from the instances of clinical relevant dynamic loading moments, or IDL's. Each IDL represents a dynamic loading cycle, and could be determined by for example the following conditions:
An IDL could be identified if the magnitude of the pressure during the dynamic loading cycle reaches for example at least 20% of the Body Weight of the patient, and the time period during which the magnitude of the pressure exceeds a chosen percentage of the Body Weight of the patient, for example 10%, during the dynamic loading cycle is greater than for example 0.1 seconds and less than for example 2.0 seconds.

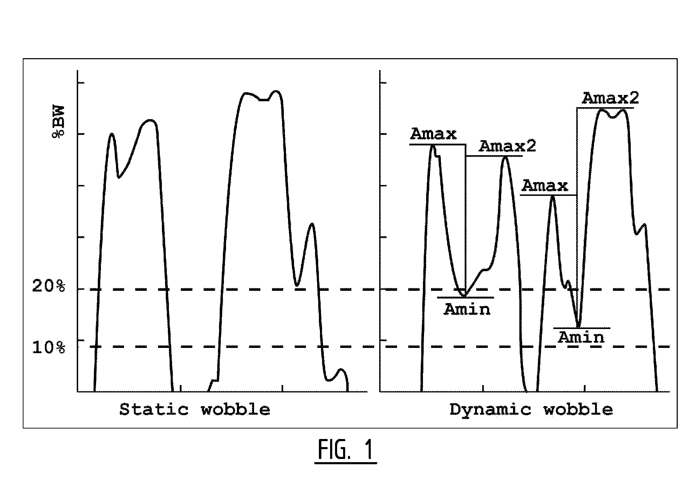

A new dynamic load cycle could start or could be considered to have started, resulting in a new IDL, if the magnitude of the pressure drops below a chosen percentage of the Body Weight of the patient, for example 10%, or if the magnitude of the pressure increases by at least for example 20% of the Body Weight of the patient from the minimum magnitude of the stress between the two dynamic load cycles after falling by at least 20% of the Body Weight of the patient from the maximum value of the previous dynamic loading cycle. This is illustrated in Figure 1. The here described definition of the IDL could be summarized as follows:
IDL: Amax > 20% BW AND
   Duration (at 10% BW) > 0.1s AND < 2.0s
New IDL: Amin < 10% BW OR
   (Amax - Amin > 20% BW AND Amax2 - Amin > 20% BW)
Where
A is the magnitude of the pressure
BW is the Body Weight of the patient

The four parameters that could be derived from the IDL's are:

### 1. Number of IDLs per unit of time (N*t⁻¹)

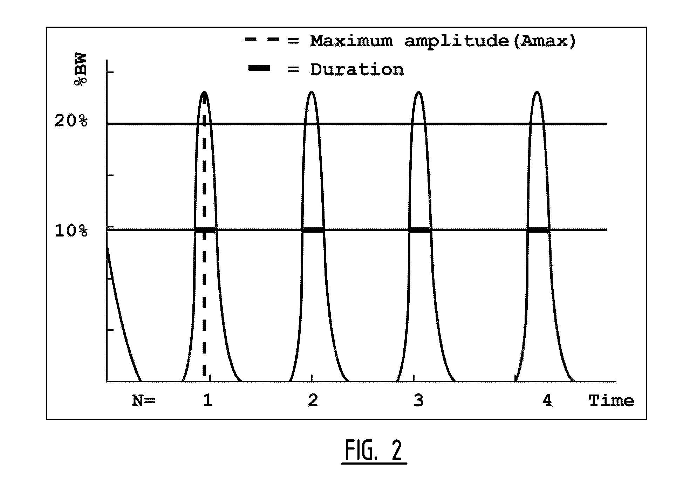

The sum of the instances of dynamic loading (IDLs) in a given period of time provide an indication of the dynamic movement pattern of the patient during that time. If this movement pattern is more dynamic, then the stimuli to bone remodelling and fracture healing could be more significantly enhanced. Figure 2 provides an example of these dynamic loading cycles with 4 IDL's.

### 2. Average amplitude of IDLs (∑A/N)

The magnitude of the pressure effects the bone responses in many ways. Relatively low pressures induce the formation of intramembranous tissue and bone (strength), and larger stresses induces the process of endochondral ossification (callus formation).

This parameter could be calculated by determining the maximum pressure (Amax) for every IDL. The use of a maximum pressure is relevant as in combination with a certain frequency, the magnitude of loading leads to bone healing or hypertrophy. Amax could be used as a target load for rehabilitation. The pressure must be above a certain threshold to induce bone formation, but if the pressure is too high it may slow down the healing or damage the fracture. Twenty percent Body Weight is equal to the minimal pressure to activate bone healing by partial weight bearing.

This parameter could be the average of the Amax of all the IDL's in a given period of time, expressed as percentage of the body weight (%BW) .

### 3. Relative Duration expressed as percentage of time (D%)

This parameter describes the percentage of the total time that the injured leg of a patient is dynamically triggered, indicative of the period during which bone healing is stimulated. The Period of one IDL could be defined as the time during which the stress exceeds 10% of the Body Weight of the patient. The duration that bone healing is triggered is the sum of all Periods of the IDL's and the relative duration is the sum of all Periods of the IDL's expressed as percentage of the total time.

### 4. Bouts of Dynamic Loading per unit of time

Partial load bearing has no proportional or linear effect on bone formation. As the duration of loading is increased, the bone formation response tends to fade as the cells become desensitized. This means that periods where loading and rest are alternated will assort a larger effect on bone healing than periods where the same amount of loading is asserted in one sequence. This results in the fourth parameter, the Period of Dynamic Loading (PDL) or bout. One bout could be established if a period of for example at least 5 minutes during which for example at least 20 IDL's take place is followed by a period of for example at least 20 minutes during which less than for example 15 IDL's take place. The number of bouts during a given period of time (such as for example one day) could provide an indication of the amount of dynamically active periods during that time, and an indication of the progress of healing.

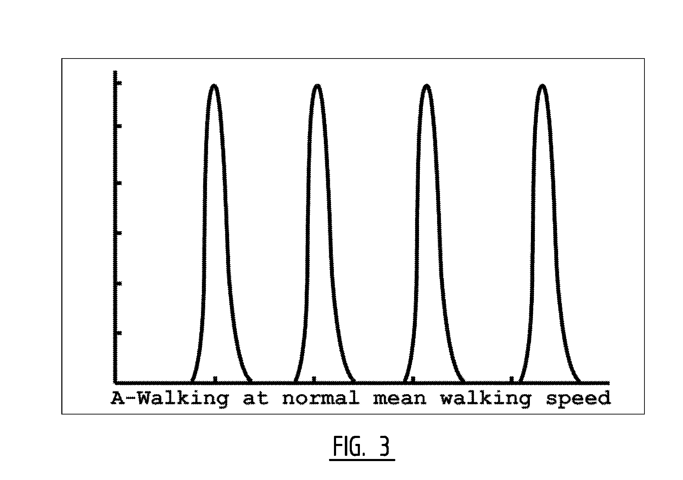

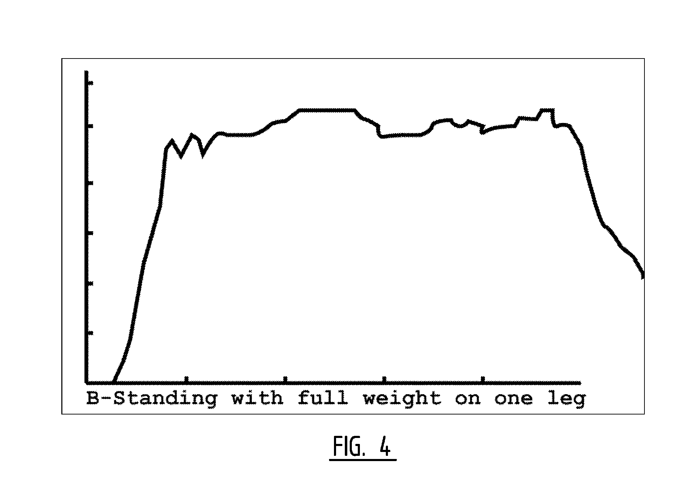

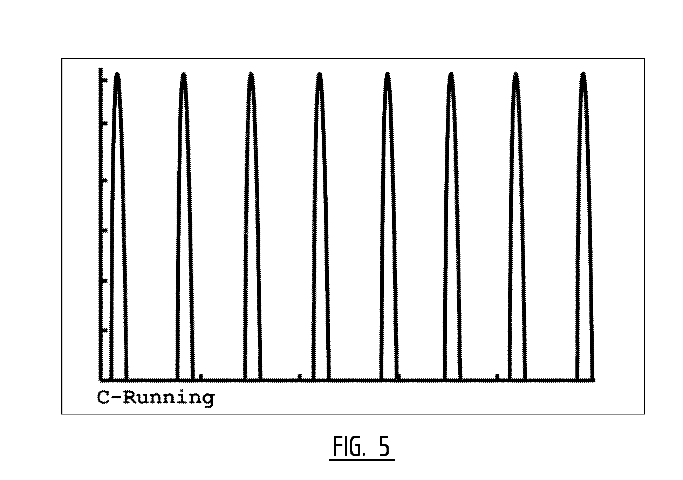

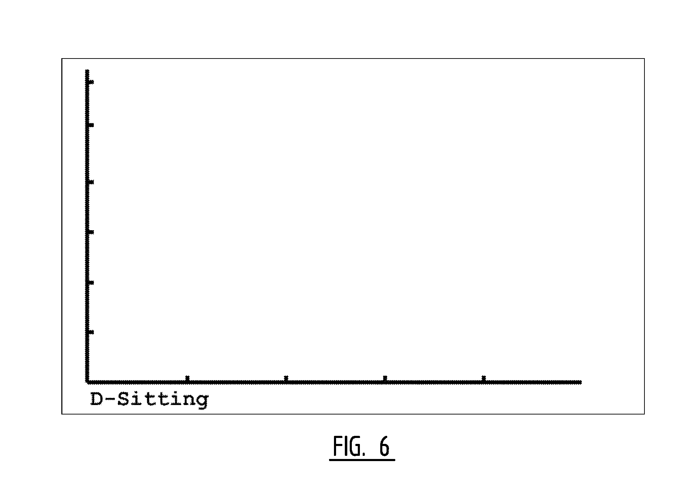

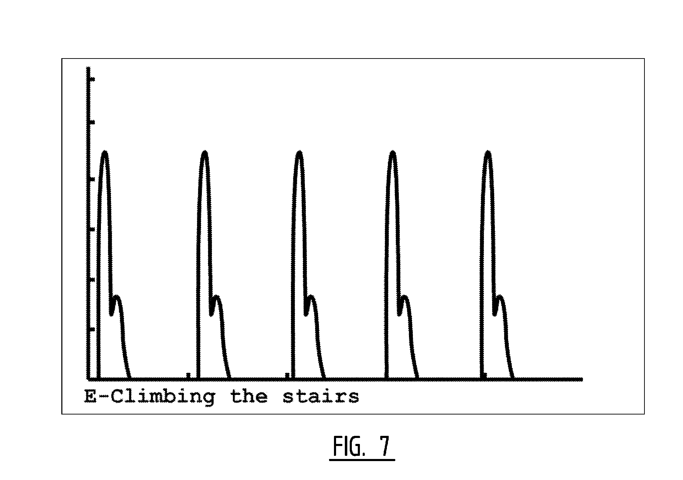

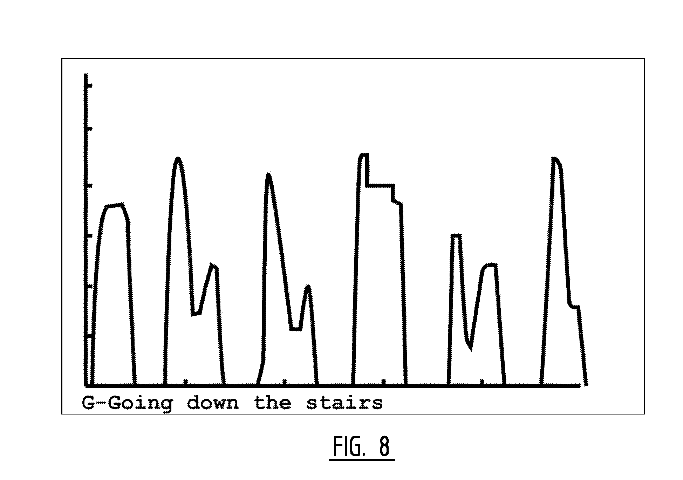

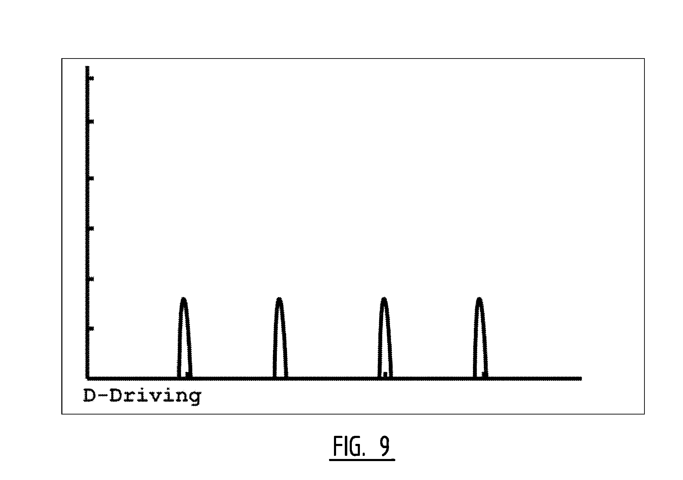

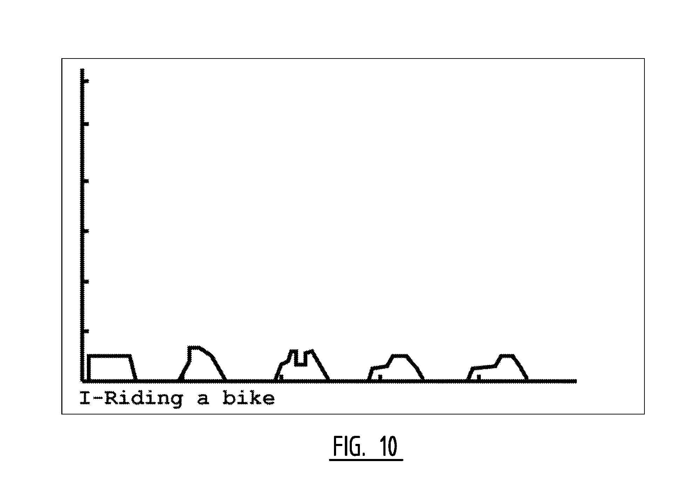

Using the example embodiment of the system, eight different activities where observed, which are shown in Figures 3 - 10. The data shows the dynamic pressure for the different activities.

The present invention is not limited to the embodiment shown, but extends also to other embodiments falling within the scope of the appended claims.

## Claims

1. Method for processing load data comprising the load measured on a lower extremity of a patient suffering from lower extremity injury, in particular a lower extremity bone injury such as a fracture, over a time period during or before rehabilitation, the method comprising selecting dynamic load data from said load data, wherein load data is selected as dynamic load data when the load temporary, within a predetermined load time period, exceeds a predetermined load threshold.

2. Method according to claim 1, wherein the load time period is 0,01 to 20 seconds, preferably 0,02 to 10 seconds, more preferably 0,075 to 5 seconds and even more preferably the load time period is 0,1 to 2 seconds.

3. Method according to claim 1 or 2, wherein the load threshold is at least 3%, more preferable at least 10%, even more preferably at least 20% of the total body weight of the patient.

4. Method according to claim 1, 2 or 3, further comprising increasing the load threshold with progressing time in the load data, in particular with rehabilitation progression.

5. Method according to any of the preceding claims 1 to 4, wherein said load data comprises axial load data having an axial component with respect to the lower extremity, in particular axial fracture load data.

6. Method according to any of the preceding claims 1 to 5, wherein the load data comprises pressure measurements under the foot, in particular under the heel, of said lower extremity.

7. Method according to any of the claims 1 to 6, further comprising the step of measuring the load on said lower extremity for providing the load data.

8. Method according to claim 7, wherein the steps of measuring and processing the load data take place in at least one portable device, preferably carried by the patient, and wherein the method further comprises the step of sending, preferably wirelessly, the dynamic load data to a remote server.

9. Method according to any of the preceding claims 1 to 8, wherein selecting dynamic load data comprises identifying instances of dynamic loading in the load data as clinically relevant data, wherein a load peak in the load data is identified as an instance of dynamic loading if the load peak exceeds the load threshold for a time period within the load time period.

10. Method according to claim 9, wherein a second load peak following a first load peak identified as an instance of dynamic loading is identified as a subsequent instance of dynamic loading if:
- the minimum load between the first and second peaks is lower than a predetermined differentiating threshold, wherein the differentiating threshold is preferably 3%, more preferably 10% of the total body weight of the patient, or;
- the difference between the minimum load between the first and second peak and each of the maximum loads of the first and second peaks is larger than the load threshold.

11. Method for establishing at least one parameter being indicative for the progress of a rehabilitation process of a patient suffering from lower extremity injury, in particular a lower extremity bone injury such as a fracture, the method comprising the steps of:
- processing load data according to claim 9 or 10;
- establishing said parameter based on the identified instances of dynamic loading, wherein the parameter is based on the number of instances of dynamic loading, the average load, preferably the average peak load, of the instances of dynamic loading, the total duration of the instances of dynamic loading, preferably the total duration of the load exceeding the load threshold, or a combination thereof.

12. Method according to claim 11, further comprising identifying active loading time periods and resting time periods from the dynamic load data, the active loading time periods being representative of a period of activity of the patient, in particular having more instances of dynamic loading per time period than the resting time periods being representative of a period of resting of the patient, wherein the parameter is established based on the number of active loading time periods followed by a resting time period.

13. Method according to claim 11 or 12, further comprising the step of outputting at least one parameter to at least one of the patient and a physician for providing feedback on the progress of the rehabilitation process.

14. System for establishing at least one parameter being indicative for the progress of a rehabilitation process of a patient suffering from lower extremity injury, in particular a lower extremity bone injury such as a fracture, the system comprising:
- a load measuring device arranged for measuring load data comprising the load measured on the lower extremity of the patient over a time period during or before rehabilitation;
- a processing device for processing the load data measured by the load measuring device into dynamic load data according to any of the claims 1 to 10, and;
- an analysing device for establishing at least one parameter according to claims 11 or 12.

15. System according to claim 14, further comprising a remote server, wherein at least one of the load measuring device, the processing device and the analyzing device is arranged to send the data and/or the parameter to the remote server, preferably wirelessly.
